Europäisches Patentamt

European Patent Office

Office européen des brevets

(11) Veröffentlichungsnummer: **0 160 292**
**A2**

(12) **EUROPÄISCHE PATENTANMELDUNG**

(21) Anmeldenummer: 85105195.3

(22) Anmeldetag: 29.04.85

(51) Int. Cl.⁴: **G 01 N 3/08**
**G 01 N 3/04**

(30) Priorität: 30.04.84 DE 3416082
29.04.85 DE 3515388

(43) Veröffentlichungstag der Anmeldung:
06.11.85 Patentblatt 85/45

(84) Benannte Vertragsstaaten:
AT BE CH DE FR GB IT LI LU NL SE

(71) Anmelder: Marek, Konrad
Gennachstrasse 10
D-8938 Buchloe(DE)

(72) Erfinder: Marek, Konrad
Gennachstrasse 10
D-8938 Buchloe(DE)

(54) Verfahren, Prüfstreifen und Vorrichtung zur Prüfung der Zugfestigkeit von Papier, Karton, Pappe u.ä.

(57) Die Erfindung beschreibt ein automatisches Zugfestigkeitsmeßgerät zur Prüfung von flächenförmigen Materialien oder von Fäden. Dazu wird eine Mehrzahl von Prüfstreifen zu einer Einheit zusammengefaßt, indem die Enden der parallel ausgerichteten Prüfstreifen untereinander verbunden werden. Das kann mittels eines Klemmrahmens, durch Ausstanzen aus einem Bogen, oder durch Einklemmen zwischen Transportbändern geschehen. Es werden Vorrichtungen zum schrittweisen Transport der Mehrstreifeneineheiten beschrieben. Zur Messung werden zwei speziell ausgebildete Meßklammertypen verwendet, die die verbindenden Teile zwischen den Prüfstreifen umgreifen bzw. umgehen können. Der Meßlammertyp mit Umgehungsteil ist durch Ausschwenken der Meßeinheit aus der Ebene der Prüfstreifen auch für Materialien mit hoher Dehnung geeignet.

FIG. 13

EP 0 160 292 A2

0160292

|

Marek, Konrad, 8938 Buchloe

Verfahren, Prüfstreifen und Vorrichtung zur Prüfung
der Zugfestigkeit von Papier, Karton, Pape u.ä.

Die Erfindung bezieht sich auf ein Verfahren zum
Prüfen der Zugfestigkeit von Papier, Pappe, Kunststoffolien u. ä. Materialien gemäß den Ansprüchen
1 bis 10 und auf Vorrichtungen zum Durchführen des
Verfahrens und unter Verwendung der Prüfstreifen
gemäß den Ansprüchen 11 bis 23.

Die Zugfestigkeitsprüfung wird an Prüfstreifen aus
dem zu prüfenden Material vorgenommen, die
vorzugsweise genormte Abmessungen aufweisen. Die
Prüfung erfolgt in Längs- und Querrichtung,
beispielsweise einer Papierbahn. So werden aus
einem Blatt bzw. einem Probebogen jeweils eine
bestimmte Anzahl, vorzugsweise 10 Prüfstreifen, in
Längsrichtung und Querrichtung entnommen.

2

Bei der Durchführung der Messung mit den bekannten Meßeinrichtungen muß jeder einzelne Prüfstreifen manuell zwischen Meßklammern des Meßgerätes eingelegt und festgespannt werden. Über beispielsweise Knopfdruck oder eine Lichtschranke wird die Zugfestigkeitsprüfung gestartet. Das Meßgerät setzt dabei den Prüfstreifen unter zunehmende Zugspannung, wobei die Meßwerte laufend registriert werden. Die Dehnungsgeschwindigkeit ist dabei meist derart justiert, daß die Zeit vom Start bis zum Zerreißen des Probestreifens 15 bis 30 Sekunden dauert. Danach werden die Klammern manuell oder automatisch zurückgesetzt und der zerrissene Prüfstreifen aus den Klammern entfernt. Die Bruchkraft, Dehnung und Dehnungsarbeit können abgelesen oder automatisch ausgedruckt und/oder die Meßergebnisse an einen Computer zwecks Auswertung übertragen werden.

Bei bekannten modernen Zugfestigkeits-Prüfgeräten laufen die meisten der vorgenannten Verfahrensschritte bereits automatisch ab. Nicht automatisiert sind dabei der erste und praktisch der letzte Verfahrensschritt, nämlich das Einlegen der Prüfstreifen zwischen die Klammern und das Entfernen der zerrissenen Prüfstreifen nach dem Prüfen. Daher bleibt der durch die Teilautomatisierung erzielte Rationalisierungserfolg bei den bekannten Vorrichtungen begrenzt, da die beiden vorerwähnten Verfahrensschritte weiterhin manuell ausgeführt werden müssen.

3

So ist in der Praxis die Arbeit mit einem modernen elektronischen Meßgerät nur unwesentlich schneller als mit einem alten, mechanischen Meßgerät, denn die Zeit zum Ablesen und Notieren des Meßergebnisses ist gering im Vergleich zu den Belastungszeiten von 10 bis 30 Sekunden, die in den Prüfnormen vorgeschrieben sind.

Aufgabe der Erfindung ist es, ein Verfahren zur Prüfung der Zugfestigkeit von Papier, Pappe o.ä. Materialien, Vorrichtungen zur Durchführung dieses Verfahrens und entsprechende Probestreifen für Verfahren und Vorrichtungen bereitzustellen, mit Hilfe deren eine vollautomatische Prüfung in einfacher und rationeller Weise durchgeführt werden kann.

Diese Aufgabe wird dadurch gelöst, daß eine Mehrzahl von Prüfstreifen an ihren Enden miteinander zu einer Mehrstreifeneinheit verbunden sind und daß das Zugfestigkeitsmeßgerät Meßklammern hat, die über die Verbindungen hinweg die einzelnen Prüfstreifen festklemmen und zum Zweck der Messung zerreißen können.

Das automatische Meßverfahren besteht darin, daß die Mehrstreifeneinheit als Handhabungseinheit verwendet wird, die mittels einer Vorschubeinrichtung, bestehend aus Führungs-, Antriebs-, Signal- und Steuerungseinrichtungen, taktweise vorgeschoben wird und daß die Meßklammern

4

einen Prüfstreifen festklemmen, ohne andere Teile der Handhabungseinheit zu berühren. Die Meßklammern setzen den Prüfstreifen unter Zugspannung und die auftretenden Zugkräfte und Dehnungen können gemessen werden. Beim nächsten Takt der Handhabungseinheit wird der zerrissene Prüfstreifen aus dem Bereich der Meßklammern entfernt.

Eine weitere Automatisierungsstufe dieses Verfahrens besteht darin, mehrere Mehrstreifeneinheiten in einem Magazin zusammenzufassen und die Vorschubeinrichtung aus diesem Magazin mit Mehrstreifeneinheiten zu versorgen. Abgesehen von der Tatsache, daß durch dieses Verfahren manuelle Arbeit, insbesondere das Einlegen und Wiederentfernen der Prüfstreifen aus den Meßklammern gespart wird, kann durch das Zusammenfassen mehrerer Probestreifen und insbesondere durch die Magazinierung der Mehrstreifeneinheiten ein örtlich und zeitlich unabhängiges, an rationellen Arbeitsabläufen orientiertes Vororganisieren der Prüftätigkeit einer unbeaufsichtigten, automatischen Zugfestigkeitsprüfmaschine vorgenommen werden.

Zur Durchführung des erfindungsgemäßen Verfahrens werden mehrere Prüfstreifen nebeneinander zumindest an ihrem einen Kopfende miteinander zu einer Einheit (Mehrstreifeneinheit) verbunden. Die Längsmittellinien der Prüfstreifen sind parallel zueinander ausgerichtet und die Vorschubrichtung

5

der Mehrstreifeneinheit ist im wesentlichen senkrecht zu diesen Längsmittellinien. Das Zusammenfassen von einer Mehrzahl von Prüfstreifen bzw. das Herstellen einer Mehrstreifeneinheit kann erfindungsgemäß in unterschiedlicher Weise durchgeführt werden. So können die Prüfstreifen nur an ihren einen Enden durch entsprechende Mittel zusammengefaßt sein. Es können jedoch auch jeweils beide Enden der im wesentlichen parallel nebeneinander angeordneten Prüfstreifen in quer zur Anordnung der Prüfstreifen ausgerichteten Mitteln zusammengefaßt werden. Die Prüfstreifen können in der Mehrstreifeneinheit eng nebeneinander liegen, nämlich nur durch Schlitze oder Schnitte getrennt. Vorzugsweise liegen sie aber durch Zwischenräume voneinander getrennt und sind nur an ihren Enden miteinander verbunden. Die Prüfstreifen können zur Bildung einer Mehrstreifeneinheit zwischen Bändern oder dgl. eingelegt werden, die jeweils paarweise an den Enden der Prüfstreifen und im wesentlichen rechtwinklig zu den Prüfstreifen verlaufen. In der einfachsten Ausführung einer Mehrstreifeneinheit kann je ein Klebstreifen über eine Reihe von Prüfstreifenenden geklebt werden.

Eine Mehrstreifeneinheit kann auch dadurch hergestellt werden, daß aus einem zu prüfenden Materialbogen mehrere Streifen derart herausgestanzt werden, daß die Kopfenden der verbleibenden Streifen über einen senkrecht zu ihrer Längsachse stehenden Verbindungssteg miteinander verbunden bleiben.

6

Die nicht herausgestanzten, verbleibenden Streifen bilden die Prüfstreifen, die zu einer leiterartigen Ausbildung zusammengefaßt sind. Durch einen einzigen Stanzvorgang werden so aus einem Probenblatt in allerschnellster Weise die benötigten 10 Prüfstreifen für die Messung bereitgestellt. Um eine bessere Festigkeit dieser Mehrstreifeneinheit zu erzielen, kann beim Stanzvorgang der Übergang von Probestreifen zu Verbindungsstegen abgerundet gestaltet werden. Die Verbindungsstege können dabei eine große Breite haben.

Die oben genannten Ausführungsformen der Mehrstreifeneinheiten haben ähnlich wie ein Papierblatt keine Steifigkeit.

Die erfindungsgemäß beste, vielseitigste und störungsunanfälligste Ausführungsform der Handhabungseinheit besteht darin, daß zwei Klemmrahmen verwendet werden, zwischen denen durch ihr Zusammenklemmen eine Mehrzahl von Prüfstreifen festgehalten wird. Ein Klemmrahmen ist vorzugsweise rechteckig ausgebildet und die Seiten, die parallel zu den Prüfstreifen liegen, sind etwas länger als die freie Prüfstreifeneinspannlänge zwischen den Meßklammern, aber höchstens gleichlang wie die Prüfstreifen.

Mit einem Klemmrahmenpaar können mehrere einzelne Prüfstreifen festgeklemmt werden oder es kann ein kompletter Probebogen festgeklemmt werden, aus dem nachträglich ein Prüfstreifenmuster ausgestanzt wird. Besonders vorteilhaft ist die Kombination einer aus einem Probebogen leiterförmig ausgestanzten Mehrstreifeneinheit (wie weiter oben bereits beschrieben) mit einem Klemmrahmenpaar zu einer Handhabungseinheit. Dadurch sind nur noch wenige Handgriffe nötig: Ausstanzen einer leiterförmigen Mehrstreifeneinheit aus einem Probebogen in einem Hub, Einlegen der Mehrstreifeneinheit in das offene Klemmrahmenpaar und Zusammenstecken des Klemmrahmenpaares.

Die Klemmrahmen können zusammensteckbar oder zueinander verschwenk- und arretierbar sein. Sie weisen jeweils an den die Prüfstreifenenden erfassenden Innenseiten Klemmeinrichtungen auf, die ein sicheres Festhalten der Prüfstreifen garantieren. Die Klemmeinrichtung kann dabei aus einer rutschfesten Auflage bestehen, die eine einfache plane Ausführung oder eine Schlauchform aufweisen kann. Durch die wahlweise Ausbildung der rutschfesten Auflagen und deren Anordnung zueinander, nämlich genau übereinander oder etwas verschoben zueinander oder winklig zueinander, kann die Aufnahme der Prüfstreifen in den Rahmen mit oder ohne Vorspannung plan ausgerichtet, oder ohne Vorspannung leicht durchhängend stattfinden. Für die Meßgenauigkeit kann eine Ausführung der Rahmen

8

mit locker durchhängenden Prüfstreifen am besten sein. Für Handhabung und Transport sind Ausführungen mit Vorspannung der Prüfstreifen günstiger. Eine den Forderungen der Prüfnormen genügende Ausführung mit genau ebener Einspannung der Prüfstreifen ohne Vorspannung dürfte nur bei Einhaltung enger Toleranzen, horizontaler Magazinierung der Klemmrahmen und vertikaler Meßanordnung erzielbar sein. Es kann auch ein Klemmrahmen gebaut werden, bei dem durch Zusammendrücken der Hauptstreben oder von Teilen davon die Prüfstreifen vom vorgespannten Zustand in einen lose durchhängenden Zustand übergeführt werden.

Um die Klemmrahmen besonders gut transportierbar und magazinierbar zu gestalten, können die Rahmen Füße aufweisen, die mit den entsprechenden Füßen eines nachfolgenden Rahmenpaares zusammenwirken.

Zur Lösung der erfindungsgemäßen Aufgabe wird weiterhin eine Vorschub- und Transporteinrichtung verwendet, die den schrittweisen Transport der Handhabungseinheiten in den Greifbereich der Meßklammern durchführt. Die Transportrichtung ist im wesentlichen senkrecht zu der Verbindungslinie der Meßklammern.

Um die Funktionen der Handhabungseinheiten mit den Funktionen der Meßklammern vereinen zu können, ist erfindungsgemäß eine Ausbildung der Meßklammern gefunden worden, die einen in einer Handhabungseinheit befindlichen Prüfstreifen

9

greifen und festklemmem können, ohne andere
Bestandteile der Handhabungseinheit zu berühren.
Dazu müssen die äußeren Teile der
Handhabungseinheit von den Meßklammern bzw. von den
Halterungen der Meßklammern umgangen werden. Die
äußeren Teile einer Handhabungseinheit sind in
diesem Zusammenhang die Teile, die sich außerhalb
der freien Einspannlänge der Prüfstreifen bzw. die
sich außerhalb des Zugbereichs der Prüfstreifen
befinden. Die äußeren Teile sind insbesondere die
Verbindungsstreifen bzw. die Hauptstreben der
Klemmrahmenpaare, die die Prüfstreifen festhalten,
oder die Transportbänder und die Abstützschienen
auf denen die Transportbänder laufen.

Die dafür beste Ausführung besteht darin, daß die
Meßklammern eine innere lichte Weite (ein Loch) von
ausreichender Größe aufweisen, die (das) das
Hindurchtreten der äußeren Teile der
Handhabungseinheiten zuläßt.
Nur bei dieser Ausführungsform der Meßklammern ist
der Transport der entsprechend ausgebildeten
Handhabungseinheiten in einfach gerichteten
Vorschubbewegungen möglich.

Eine andere Ausführungsform der Meßklammern besteht
darin, daß die Öffnung der Meßklammer so angeordnet
ist, daß, in Vorschubrichtung gesehen, die
Prüfstreifen von vorne oder in einer analogen
Ausbildung von hinten in die Öffnung bis zum
Anschlag einfahren können, und daß die Halterungen
der Meßklammern so ausgebildet sind, daß sie die
äußeren Teile der Handhabungseinheiten z.B.

bogenförmig umgehen. Nach der Messung können die zerrissenen Streifen in Gegenrichtung wieder ausgefahren werden. Vor der nächsten Messung ist aber mindestens eine zusätzliche Bewegung der Handhabungseinheit relativ zu den Meßklammern notwendig, die im wesentlichen senkrecht zu der Ebene ist, die von der Vorschubrichtung und der Prüfstreifenlängsrichtung aufgespannt wird.

Dieser Meßklammertyp erfordert zwar eine zusätzliche Hubbewegung, er ermöglicht aber eine Prüfgeräteausführung für plastische Materialien oder solche mit hoher Elastizität, wie z.B. bei der Prüfung von Gummifäden.

Bei einer solchen Prüfgeräteausführung wird der Prüfstreifen oder dgl. von den Meßklammern festgespannt, aus der Ebene der Abstützplatte herausgehoben oder geschwenkt und dann gemessen. Dadurch ist ein langer Dehnungsweg möglich.

Die Ausbildung der Transporteinrichtung kann bei der Verwendung von Klemmrahmenpaaren, die eine Eigensteifigkeit und Stabilität haben, relativ einfach gestaltet werden. Sie kann aus Führungsschienen, Führungs- und Antriebsrollen, Positonsdetektoren und einer einfachen Vorschubsteuerung bestehen.

Neben der Transporteinrichtung kann die Prüfvorrichtung zur weiteren Erhöhung des Automatisierungsgrades zumindest ein Magazin und eine Entnahmeeinrichtung für Handhabungseinheiten aufweisen. Vorteilhaft ist auch ein Aufnahmemagazin für die Handhabungseinheiten, die die Meßstation durchlaufen haben. Die Magazine können dabei zur nebeneinander aufrechtstehenden Aufnahme der Handhabungseinheiten ausgebildet sein (horizontale Magazinierung). Sie können aber auch zur übereinander gestapelten Aufnahme horizontal liegender Handhabungseinheiten ausgelegt sein (vertikale Magazinierung). Die Vereinzelungseinrichtung ist derart ausgebildet, daß aus den Magazinen jeweils eine Handhabungseinheit entnommen wird.

Die Vereinzelungsvorrichtung kann eine Greifeinrichtung sein, die eine Handhabungseinheit abhebt oder bei vertikaler Magazinierung den ganzen Stapel mit Handhabungseinheiten derart anhebt, daß die zuunterst liegende Handhabungseinheit freigesetzt wird und beispielsweise auf einer angetriebenen Rollenbahn den Meßklammern zugeführt werden kann.

In gleicher Weise wie die Vereinzelungseinrichtung kann eine weitere Einrichtung an dem Aufnahmemagazin für die geprüften Teile vorgesehen werden, die die Handhabungseinheiten von unten in den angehobenen Stapel schieben oder von oben in das Magazin einbringen.

Bei einer Prüfvorrichtung für die Verwendung von sehr langen Probebahnen als Handhabungseinheiten wird vorzugsweise eine Magazintrommel benutzt. Die Magazintrommel kann zum Spannen der abgewickelten Probebahn mit einer Bremseinrichtung versehen sein.

In einer vorteilhaften Ausführung des Prüfgeräts befindet sich zwischen Magazintrommel und Meßklammern eine Stanzeinrichtung, die in einer noch ungestanzten Prüfbahn Zwischenstreifen ausstanzt und so eine leiterförmige Anordnung der Prüfstreifen erzeugt.

Eine andere Ausbildung der Transporteinrichtung ist bei der Verwendung von Handhabungseinheiten ohne Eigenstabilität notwendig. Beispielsweise ein plattenförmiges Abstützungselement, Führungsrollen, Antriebs-, vorzugsweise Zugrollen bzw. Zugrollenpaare, Positions- und Lagedetektoren, eine Vorschubsteuerung und eine Positionsregelung seitlich zur Vorschubrichtung zur fluchtenden Ausrichtung der Handhabungseinrichtungen.
Für eine nur geringen Herstellungsaufwand erfordernde Lösung werden erfindungsgemäß zwischen Rollen aufgespannte, umlaufende Transportbänder benutzt. Die Prüfstreifen, bzw. Mehrstreifeneinheiten werden zwischen den Bändern eingelegt. Die Bänder sind so angeordnet, daß sie die Enden eines jeden Prüfstreifens außerhalb seines Meßbereichs erfassen. Das Festhalten der Prüfstreifen wird durch Zusammenpressen der Bänder erreicht, wobei die Bänder auf einer Abstützplatte oder auf Abstützschienen geführt werden und durch Andruckrollen, oder Eigengewicht zusammengepreßt werden können. Vorzugsweise werden die Bänder durch durch das Zusammenwirken von Bandspannung und Führung auf einer gekrümmten Bahn zusammengepreßt. Dazu wird eine gekrümmte Abstützplatte oder zwei

13

gekrümmte Abstützschienen verwendet, auf denen die Bänder laufen. Die erforderliche Zusammenpreßkraft kann durch den Krümmungsradius und die Bandspannung eingestellt werden.

Zum Einlegen der Prüfstreifen werden die beiden oberen Bänder hochgekklappt. Durch das Zuklappen der Bänder werden die Prüfstreifen zu Mehrstreifeneinheiten zusammengefaßt. Es können aber auch bereits vorgefertigte Mehrstreifeneinheiten in die Bänder eingelegt werden.

Zum Ausrichten der Prüfstreifen kann auf der Abstützplatte ein Anschlag oder ein optisch gut sichtbares Streifenmuster aufgebracht werden.

Um bei dieser Bauweise der Handhabungseinrichtungen und Transporteinrichtungen eine größere Anzahl von Prüfstreifen unterzubringen kann eine relativ lange gekrümmte Abstützplatte verwendet werden.

Durch die beschriebenen Merkmale wird die vollautomatische Integrierbarkeit der Zugbelastungsprüfung erreicht.

Um bei der Zugfestigkeitsmessung, insbesondere bei der Messung der Bruchdehnung und daraus abgeleiteter Größen, ein exaktes Ergebnis zu erhalten, wird in Normen, z.B. DIN 53112, gefordert, daß jeder Prüfstreifen zu Beginn der Messung genau in der Ebene liegt, die von den geschlossenen Backen der Meßklammern aufgespannt

/4

wird und daß der Prüfstreifen keiner Vorspannung
unterliegt.

In der Praxis gibt es verschiedene Probleme, die
die exakte Einhaltung dieser Bedingungen
verhindern. Die Prüfstreifen können leicht gekrümmt
sein und nicht genau plan liegen. Bei der manuellen
Prüfung macht jeder Prüfer geringfügig
unterschiedliche Handgriffe beim Ausrichten und
Einspannen der Prüfstreifen , was zu
unterschiedlichen Vorspannkräften führt. Bei
horizontaler und bei vertikaler Anordnung der
Meßklammern können Unterschiede durch Eigengewicht
und/oder Handhabung bestehen.
Bei einem automatischen Prüfgerät mit
Handhabungseinheiten können diese Probleme auch
auftreten.

Eine Lösung der Probleme besteht darin, den
jeweiligen Prüfstreifen im Bereich seiner freien
Einspannlänge zwischen zwei Platten leicht
zusammenzupressen, in der Ebene der
Meßklammerbacken auszurichten und erst dann
festzuspannen.

Eine andere Lösung ist dann möglich, wenn Mittel
vorhanden sind, um das Kraft-Dehnungsdiagramm
grafisch oder per Computer auszuwerten. Der
Ausgangspunkt der Dehnungsmessung wird durch
lineare Extrapolation des geraden Teils der Kraft-
Dehnungskurve zur Nullinie der Kraftskala gewonnen.
Für die Dehnungsarbeit wird ebenfalls ein linearer
Verlauf im Anfangsbereich angenommen. Dadurch wird

*15*

das Ergebnis der Bruchdehnung weitgehend unabhängig von den Anfangsbedingungen bei der Kraft-Dehnungsmessung. Die Abweichungen von der Linearität im Anfangsbereich des Kraft-Dehnungsdiagramms können durch Abweichungen von den in der Norm geforderten Einspannbedingungen erklärt werden: Ein flacher Anstieg entsteht durch das Ausziehen von Durchhang und Krümmungen des Prüfstreifens; die dabei gemessene Kraft entspricht nicht der Zugkraft, sondern der zu überwindenden Biegekraft. Ein steilerer Anstieg entsteht bei vorgespannten Prüfstreifen. Nach dieser Methode lassen sich auch vorgespannte oder etwas durchhängende Prüfstreifen genau messen.

Bei Verwendung von Klemmrahmenpaaren in einem automatischen Prüfgerät können noch zwei weitere Meßgenauigkeitsprobleme auftreten.

Das Stück eines Prüfstreifens, das sich zwischen der Einspannstelle der Meßklammer und der Einspannstelle des Klemmrahmens befindet, kann eine Druckkraft auf die Meßklammer übertragen und das Meßergebnis verfälschen.

Durch Ausknicken bzw. Durchbiegen dieses Prüfstreifenstückes kann die übertragene Druckkraft verkleinert werden. Das Durchbiegen kann z.B. dadurch erreicht werden, daß die Hauptstreben eines Klemmrahmens beweglich ausgeführt werden, so daß sie vor der Messung in Prüfstreifenlängsrichtung etwas zusammengedrückt werden können.

*16*

Das zweite Problem kann bei der Verwendung von Klemmrahmen, die die Prüfstreifen vorspannen, auftreten.Die Klemmeinrichtungen an den Innenseiten der Hauptstreben können elastisch durchfedern und diese Federkraft kann als Zugkraft durch das Prüfstreifenstück auf die Meßklammer übertragen werden. Dieses Problem wird durch die Verwendung von Klemmrahmen mit leicht durchhängenden Prüfstreifen gelöst.

Eine andere Möglichkeit besteht darin, daß Klemmrahmen verwendet werden, bei denen die Prüfstreifen vom vorgespannten in einen durchhängenden Zustand übergeführt werden. Das automatische Meßverfahren läuft dann am besten so ab:

Vorschubschritt der Handhabungseinheit, Prüfstreifen ist in der Handhabungseinheit vorgespannt; Greifen und Festklemmen des Prüfstreifens durch die Meßklammern; Entspannen des oder der Prüfstreifen in der Handhabungseinheit durch z.B. Zusammendrücken der Hauptstreben der Klemmrahmen, so daß eines oder beide der kurzen Prüfstreifenstücke, die sich zwischen Klemmrahmen und Meßklammereinspannstelle befinden, durchhängen kann bzw. können;
Start der Zugmessung; Zerreißen des Prüfstreifens; Lösen und Zurücksetzen der Meßklammern; die restlichen Prüfstreifen wieder in den vorgespannten Zustand versetzen; nächster Vorschubschritt.

Nachfolgend wird die Erfindung anhand von Ausführungsbeispielen unter Bezug auf die Zeichnung näher beschrieben. Es zeigt:

Fig. 1 eine Handhabungseinheit mit Klemmrahmen in auseinandergezogener Darstellung;

Fig. 2 ein Detail des Klemmrahmens nach Fig. 1, eine Klammer in zwei Ansichten zeigend;

Fig. 3 eine bewegliche Ausführungsform der Klemmeinrichtung zwischen den Rahmen der Handhabungseinheit nach Fig. 1, in Seitenansicht und Draufsicht;

Fig. 4 bis 9 Ausführungsformen der Klemmeinrichtung zwischen den Rahmen der Handhabungseinheit nach Fig. 1;

Fig. 10 eine Handhabungseinheit in weiterer Ausbildungsweise, in ausgestanzter Form;

Fig. 11 eine Handhabungseinheit nach Fig. 10 mit abgerundeten Ecken;

Fig. 12 eine Handhabungseinheit in dritter Ausführungsform mit Klebebändern als Halte- oder Verbindungsstreifen;

Fig. 13 eine schematische Darstellung eines Teiles einer Prüfeinrichtung mit vertikalen Meßklammern;

Fig. 14 Meßklammern mit innerer lichter Weite und eine Hilfseinrichtung zum Ausrichten der Prüfstreifen;

Fig. 15 Meßklammern mit innerer lichter Weite und Hilfsbügeln in Seitenansicht;

Fig. 16 Meßklammern mit gebogener Zugstrebe in Seitenansicht;

Fig. 17 Meßklammern nach Fig. 16 mit gebogener Zugstrebe in Vorderansicht;

Fig. 18 ein horizontales Magazin für Handhabungseinheiten nach Fig. 1;

Fig. 19 ein vertikales Mehrfach-Magazin für Handhabungseinheiten nach Fig. 1;

Fig. 20 eine schematische Darstellung einer Prüfeinrichtung für Handhabungseinheiten nach Fig.1 mit vertikalen Magazinen nach Fig. 19

Fig. 21. eine schematische Darstellung einer Prüfeinrichtung in zweiter Ausführungsweise für lange Handhabungseinheiten nach den Fig. 10 bzw. 12;

Fig. 22 eine schematische Darstellung einer Magazintrommel der Vorrichtung nach Fig. 21;

Fig. 23 eine schematische Darstellung einer Doppelmagazintrommel der Vorrichtung nach Fig. 21;

/9

Fig. 24 eine teilweise Perspektivansicht auf die Prüfeinrichtung nach Fig. 21;

Fig. 25 eine weitere teilweise Perspektivansicht der Vorrichtung nach Fig. 21;

Fig. 26 eine perspektivische Darstellung der wesentlichen Teile einer Prüfeinrichtung in dritter Ausführungsweise für Handhabungseinheiten nach Fig.11;

Fig. 27 ein Kraft Dehnungsdiagramm mit grafischer Korrektur des Dehnungsausgangspunktes;

Fig. 28 ein Prüfgerät mit gekrümmter Abstützplatte.

Fig. 29 eine Seitenansicht eines Prüfgeräts mit längeren Bändern zur Aufnahme einer größeren Anzahl von Prüfstreifen.

Fig. 30 ein Prüfgerät mit ausschwenkbaren Meßklammern für Materialien mit hoher Dehnfähigkeit.

20

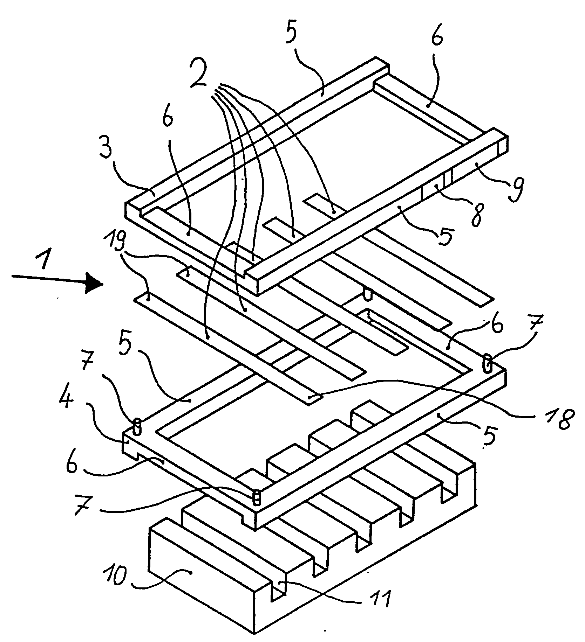

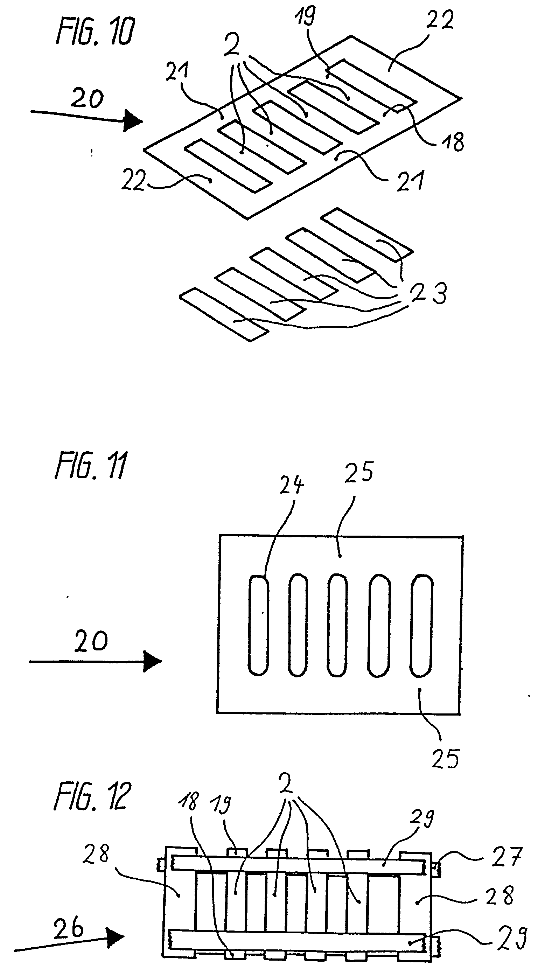

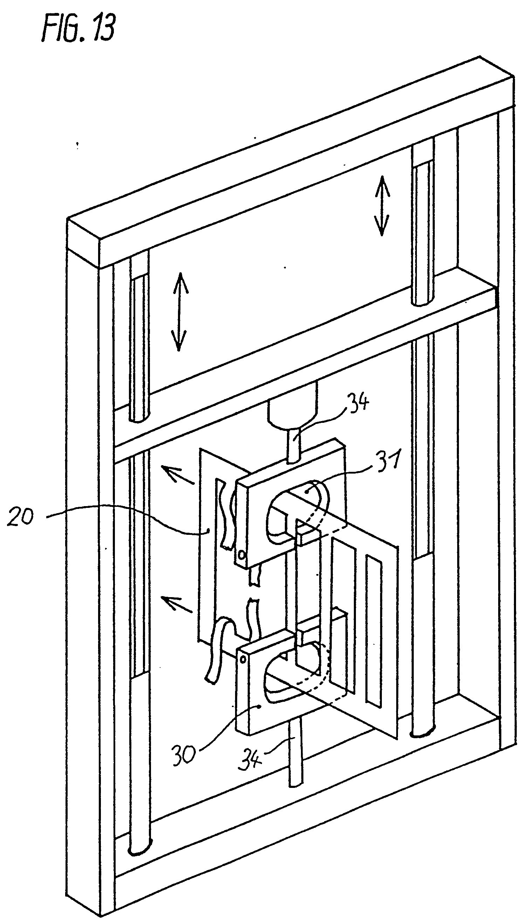

Fig. 1 zeigt eine Handhabungseinheit 1, in welcher Prüfstreifen 2 von einem Klemmrahmen, bestehend aus einem oberen Rahmen 3 und einem unteren Rahmen 4, zusammengehalten werden. Statt der Prüfstreifen 2 kann in dem Klemmrahmenpaar eine den Fig. 10 oder 11 entsprechend ausgebildete Mehrstreifeneinheit eingeklemmt werden, oder ein ganzer Probebogen eingeklemmt werden, welcher später zu Streifen geschnitten bzw. gestanzt wird. Jeder Rahmen 3, 4 besteht aus zwei Hauptstreben 5 und zwei Seitenstreben 6. Die Seitenstreben 6 sind etwas breiter als die Prüfstreifen, damit eine im Meßgerät vorgesehene Lichtschranke ein Signal abgeben kann, das von dem der Prüfstreifen unterscheidbar ist. Kurze Paßstifte 7 sind eine Hilfe, damit die Rahmen beim Zusammenklemmen richtig justiert werden. Die Länge der Prüfstreifen ist dabei so zu bemessen, daß deren Enden 15 vorzugsweise maximal 1 cm über den Rahmen hinausstehen bzw. -hängen. Die lichte Weite 31 der Meßklammern 30 ist so groß zu wählen und der Öffnungsweg der beweglichen Klemmbacken 32 muß so groß sein, daß weder der Rahmen noch die Prüfstreifenenden anstoßen. Zur Identefizierung der eingeklemmten Prüfstreifen kann an dem Rahmen ein Schriftfeld 8 oder eine Magnetkarte 9 vorgesehen sein.

Zum Einlegen von Einzelprüfstreifen ist es vorteilhaft, dem unteren Rahmen eine Schablone 10 zu unterlegen, die parallele Nuten 11 aufweist, die zumindest die Breite der Prüfstreifen 2 haben. Die Schablone dient dazu, die Prüfstreifen parallel und rechtwinklig zum Rahmen 3, 4 auszurichten.

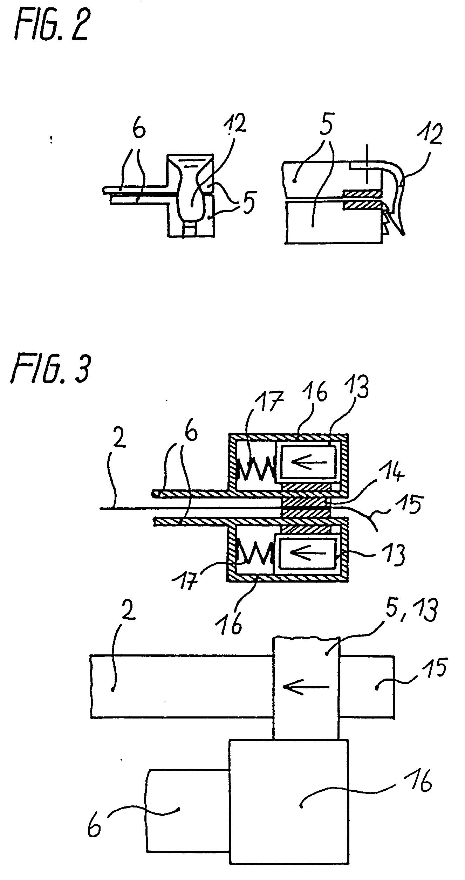

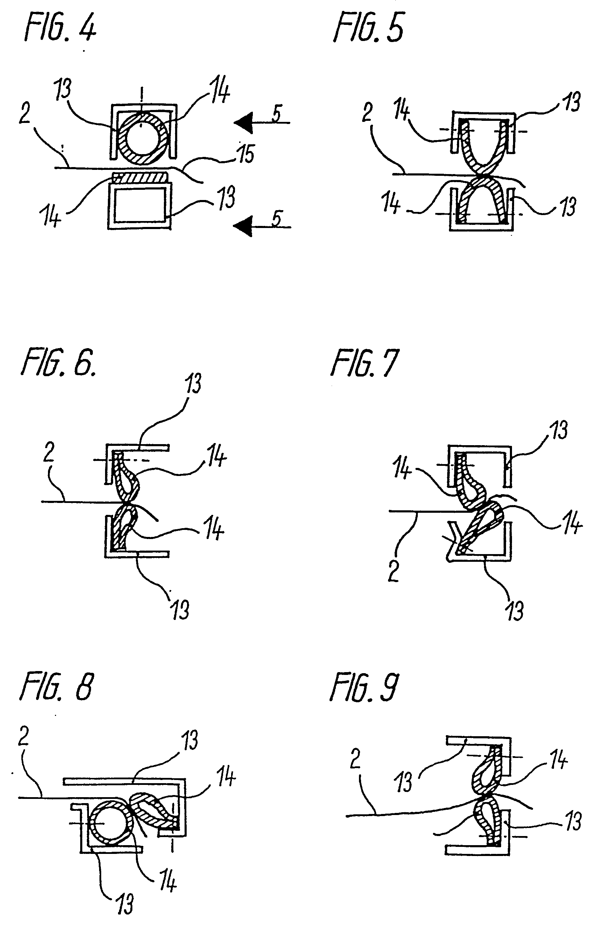

Wie Fig. 2 zu entnehmen ist, sind die Rahmen 3, 4 durch lösbare Klammern 12 zusammengehalten, die beim Zusammensetzen und Festdrücken der Rahmen 3, 4 von selbst einrasten. Die Abmessungen und die Anordnung der Klammern 12 sind dabei vorzugsweise derart auszuwählen, daß sie maximal 1 cm über den Querschnitt der zusammengeklemmten Hauptstreben 5 hinausragen. Die Klammern 12 befinden sich vorzugsweise am oberen Rahmen, da dann mit einem einzigen Handgriff die Klammern entriegelt werden können und dann der obere Rahmen aufgeklappt werden kann. Die Prüfstreifen 2 bzw. ein Probebogen werden zwischen den Innenseiten der Hauptstreben 5 festgeklemmt. Wie aus Fig. 4 zu entnehmen ist, besteht eine Hauptstrebe 5 aus einer kastenförmigen, U-förmigen oder winkelförmigen Schiene 13, die der Konstruktion Steifigkeit verleiht, und aus einem elastischen Klemmteil bzw. einer elastischen Klemmleiste 14 mit weicher gummiartiger Oberfläche, das die Prüfstreifen 2 festhält. Von besonderem Vorteil ist, wenn die elastische Klemmleiste ein geschlossener oder aufgeschnittener Gummischlauch ist. Für den Aufbau und das Zusammenwirken der elastischen Klemmleisten einer oberen und einer unteren Hauptstrebe 5 gibt es verschiedene Ausführungsformen, wie aus Fig. 4 bis 9 ersichtlich ist. Fig 4 und 5 zeigen Ausführungsformen, die keine Vorspannung in den Prüfstreifen 2 oder den Probebögen erzeugen. In den Figuren 6 bis 8 sind Ausführungsbeispiele dargestellt, in welchen die Prüfstreifen 2 vorgespannt werden.

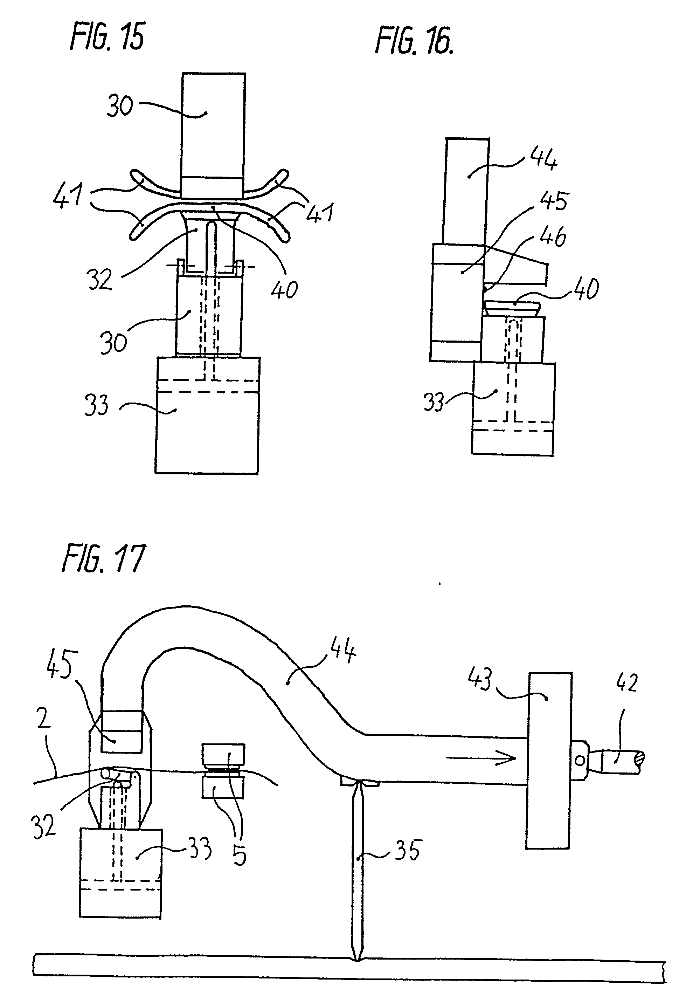

Fig. 9 zeigt eine Klemmeinrichtung, die die Prüfstreifen ohne Vorspannung leicht durchhängend festklemmt. Bei dieser Ausführung der Handhabungseinheit ist es möglicherweise von Vorteil, wenn seitlich an den Meßklammern des Prüfgeräts leicht gebogene Hilfsbügel 41 angebracht sind, wie in Fig. 15 dargestellt. Dadurch wird das Einfahren des Prüfstreifens in die Meßklammern erleichtert.

Die Klemmeinrichtung eines Klemmrahmenpaares, bei dem die Prüfstreifen von einem vorgespannten in einen durchhängenden Zustand überführt werden können, ist in Fig. 3 dargestellt (Frontansicht und Draufsicht). Die Schiene 13 einer Hauptstrebe 5 ist an ihren Enden in Gehäusen 16 beweglich gelagert. Die in den Klemmleisten 14 eingeklemmten Prüfstreifen 2 werden von Federn 17 unter Vorspannung gehalten.

Die notwendigen Klemmkräfte können durch Materialauswahl, -dicke, und -form der elastischen
Klemmleisten erzielt werden. Die gewünschten
Vorspannkräfte können im wesentlichen durch Lage
und Winkel der elastischen Bauteile festgelegt
werden.

Fig. 10 zeigt eine weitere Handhabungseinheit 20,
die aus einem Prüfbogen in einem Stück
herausgestanzt ist. Die Handhabungseinheit 20 weist
Prüfstreifen 2, an deren Enden Haltestreifen 21
verlaufen, und Seitenstreifen 22 auf. Die
Zwischenräume zwischen den Prüfstreifen 2 sind
durch Herausstanzen von Streifen 23 hergestellt
worden. Die beiden Seitenstreifen 22 weisen eine
größere Breite als die Prüfstreifen 2 auf, so daß
sie für die Lichtschranke am Meßgerät erkennbar
sind. Dieses aus dem Probebogen ausgestanzte
Prüfstreifengitter kann als selbständige Einheit
verwendet werden. Es kann aber auch in dem
Klemmrahmen 3, 4 nach Fig. 1 eingespannt werden.
Wie in Fig 11 dargestellt, kann es vorteilhaft
sein, die scharfen Ecken in den Prüfstreifengittern
zu vermeiden und den Ecken Abrundungen 24
zuzuordnen.

In Fig.12 ist eine weitere Handhabungseinheit 26
dargestellt, bei welcher die Prüfstreifen 2 mit
ihren Enden auf Klebebändern 27 aufgeklebt sind.
Anfang und Ende dieser Handhabungseinheit bilden
zwei aufgeklebte Seitenstreifen 28. Die
Seitenstreifen 28 sind etwas breiter als die

24

Prüfstreifen 2 und können aus steiferem Material bestehen. Zur besseren Haltbarkeit des ganzen Gebildes können von oben noch einmal zwei Klebebänder 29 aufgebracht werden. Diese Handhabungseinheit 26 kann auch zu langen Probebahnen zusammengesetzt sein, die eine sehr große Anzahl von Prüfstreifen 2 zusammenschließt.

Fig. 13 zeigt einen Teil eines Meßgeräts mit senkrecht angeordneten Meßklammern 30, durch die eine Handhabungseinheit 20 hindurchgeführt wird. Die Meßklammern 30 weisen eine entsprechende lichte Weite 31 auf, zum ungehinderten Hindurchführen der Hauptstreben 5 bzw. der Haltestreifen 21, 25, 27.

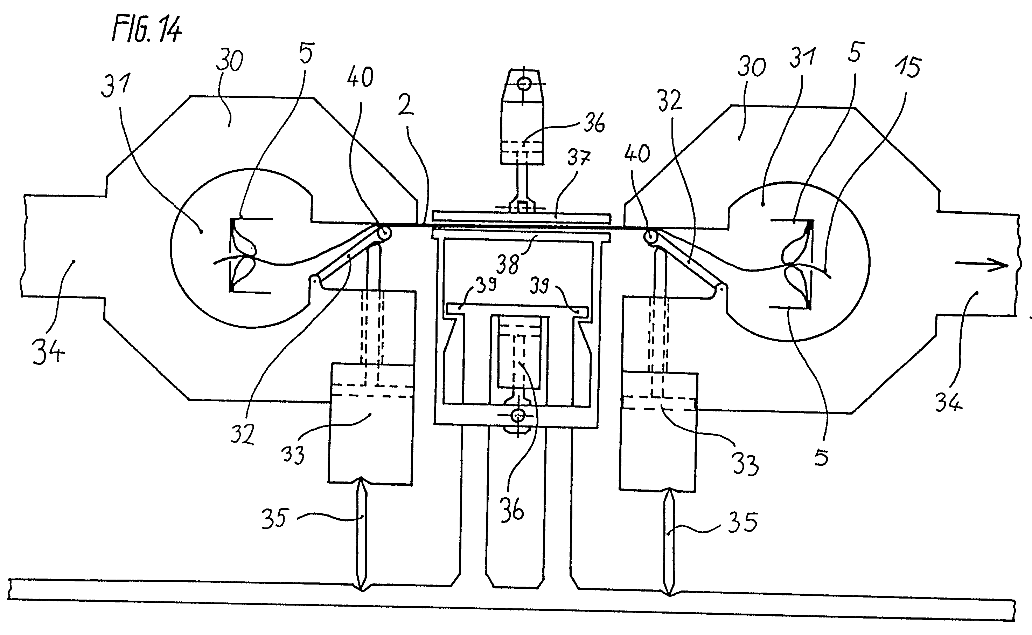

Fig.14 zeigt Meßklammern 30 mit innerer lichter Weite 31 und eine Hilfseinrichtung 37, 38 zum Ausrichten der Prüfstreifen. Die innere lichte Weite 31 muß von ausreichender Größe sein, damit die Hauptstreben 5 ungehindert hindurchtreten können. Auch die Prüfstreifenenden 15 sollen nicht anstoßen. In dieser Figur ist ein Klemmrahmen für lose durchhängende Prüfstreifen 2 verwendet worden. Vor dem Festklemmen des Prüfstreifens 2 mit den beweglichen Klemmbacken 32, die z.B. durch pneumatische Zylinder 33 betätigt werden und die einen zylinderförmigen Klemmkörper 40 tragen, wird der Prüfstreifen von einer Hilfseinrichtung ausgerichtet. Dazu dient eine untere Ausrichtplatte 38, die z.B. von einem pneumatischen

25

Betätigungszylinder 36 angehoben wird. Die Lage der Ausrichtplatte 38 wird durch die genau justierten Anschläge 39 festgelegt. Eine obere Ausrichtplatte 37 kann vorgesehen werden, um den Prüfstreifen 2 leicht anzupressen. Zur Messung werden die Ausrichtplatten zurückgezogen und der Prüfstreifen durch die Meßklammern 30 und ihre Zugstreben 34 unter Zugspannung gesetzt. Die Meßklammern können durch Stützen 35 abgestützt bzw. reibungsfrei gelagert werden.

Fig. 15 zeigt Meßklammern mit seitlich angebrachten Hilfsbügeln 41 zum leichteren Einführen der Prüfstreifen. Der untere Hilfsbügel 41 ist an dem Klemmkörper 40 angebracht und bewegt sich mit der beweglichen Klemmbacke 32 mit.

Fig. 16 zeigt eine Meßklammer in zweiter Ausführungsform mit gebogener Halterung 44 in Seitenansicht. Erkennbar sind die Backen der Meßklammer 45 und der Anschlag 46 für die Prüfstreifen.
In Fig. 17 ist die Meßklammer nach Fig. 16 in Vorderansicht dargestellt. Für die Funktionsweise ausschlaggebend ist die gebogene Ausführung der Zugstrebe (Halterung) 44. Die Zugstrebe 44 darf die Hauptstreben 5 des Klemmrahmenpaares nicht berühren. Die Biegung der Zugstrebe ist vorzugsweise so ausgeführt, daß die Spannvorrichtung 42 und die Kraftmeßzelle 43 bei der Messung fluchtend zum Prüfstreifen 2 ausgerichtet sind, der mittels der beweglichen Klemmbacke 32 festgeklemmt wird. Zum Festklemmen des Prüfstreifens dient die Betätigungsvorrichtung 33, die z.B. ein Pneumatikzylinder ist. Das gesamte Bauteil kann von einer (oder mehreren) Stütze(n) 35 reibungsfrei abgestützt werden.

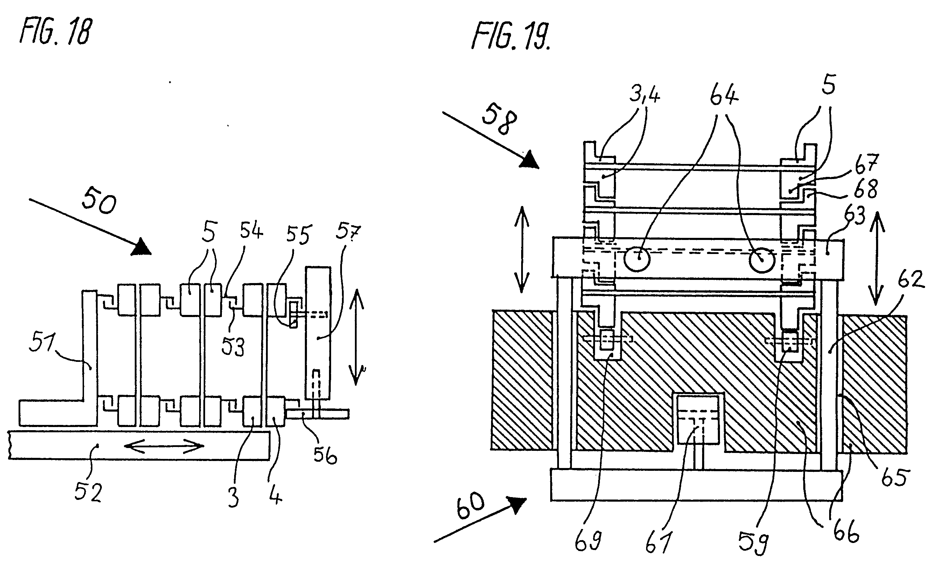

Fig. 18 zeigt ein horizontales Magazin 50 für in Klemmrahmen 3, 4 zusammengefaßte Proben. Die Hauptstreben 5 der Klemmrahmen sind mit ineinandergreifenden Füßen 53, 54 versehen. Für ein Meßgerät mit senkrecht angeordneten Meßklammern, wie in Fig. 13 dargestellt, ist es vorteilhaft, die Rahmen 3, 4 nebeneinander und stehend auf einem beweglichen Tisch 52 zu lagern. Zum Absichern gegen Umfallen ist neben dem letzten Rahmen eine Stütze 51 auf dem Tisch 52 aufgestellt. Der erste Transportrahmen wird von einem senkrecht beweglichen Träger 57 aufgenommen, nach Zurückziehen des Tisches 52 etwas abgesenkt und von am Träger 57 vorgesehenen Rollen 55 und 56 in Richtung der Meßklammern bewegt. Mittels mehrerer Führungs- und Antriebsrollen vor und nach den Meßklammern wird der Rahmen taktweise durch die Meßklammern hindurchbewegt und die Prüfstreifen werden gemessen. Der Vorschub bei der Bewegung der Rahmen durch die Meßklammern kann von Lichtschranken gesteuert werden, die die Lage der Prüfstreifen 2 erkennen können. Nach der Messung können die Rahmen in einer ähnlichen Anordnung (horizontales Magazin) wieder aufgereiht werden.

Fig. 19 zeigt ein vertikales Magazin 58, bei welchem die Rahmen 3, 4 waagrecht liegend übereinander gestapelt sind. Diese Magazinierung ist vorteilhaft insbesondere für Meßgeräte mit waagrecht angeordneten Meßklammern. Die Rahmen weisen in den Hauptstreben 5 ineinandergreifende Füße 67 und 68 auf, die einer gleichzeitigen vertikalen Stapelung dienen.

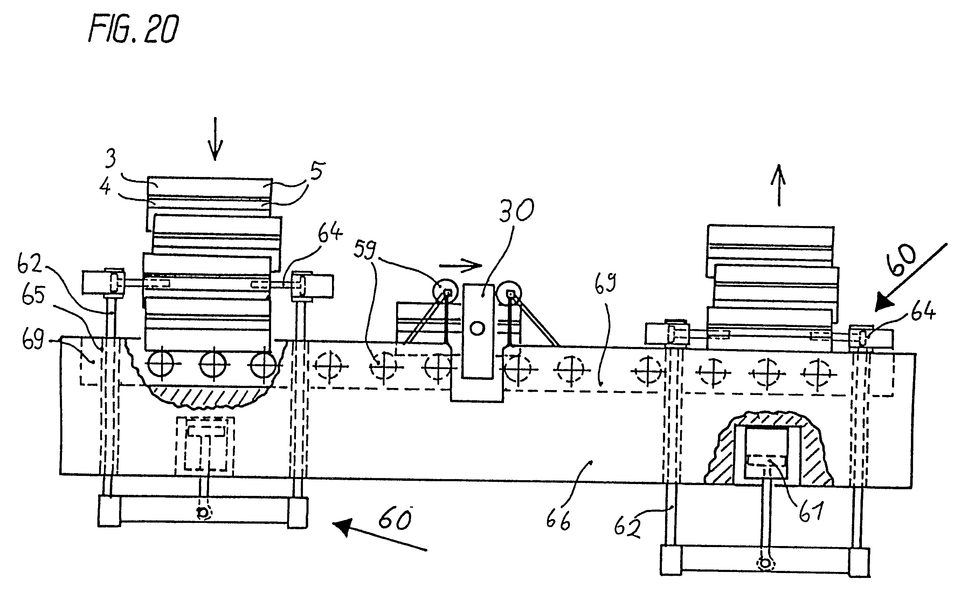

Wie insbesondere aus Fig. 20 zu ersehen ist, ist eine Vereinzelungsvorrichtung 60 vorgesehen, die eine Betätigungseinheit 61 für die Hubbewegung, in Führungen 65 verlaufende Stützen 62, Tragarme 63 und daran befestigte Eingreifeinrichtungen 64 aufweist. Die Eingreifeinrichtung 64 kann unter Seitenstreben 6 der Klemmrahmenpaare eingreifen. Die Eingreifvorrichtung 64 muß zuerst zwischen den zwei untersten Handhabungseinheiten zwischengreifen, dann hebt die Hubvorrichtung 61 die Vereinzelungseinrichtung 60 und den daraufliegenden Handhabungseinheiten-Stapel etwas an. Dadurch wird die unterste Handhabungseinheit frei und kann von Antriebsrollen 59 oder von Antriebsbändern zu den Meßklammern 30 hinbewegt werden. Die Führung der Handhabungseinheit wird durch Nuten 69 im Tisch 66 erreicht. Selbstverständlich können auch andere Führungs- und Antriebshilfen verwendet werden. Die Vereinzelungseinrichtung 60 setzt dann den restlichen Stapel auf die Antriebsrollen 59 ab, die Eingreifvorrichtung 64 fährt zurück, geht in ihre Ausgangsstellung und fährt unter dem von unten zweiten Rahmen wieder ein. Die freigesetzte Handhabungseinheit durchläuft taktweise die Meßklammern 30 und wird nach dem Durchlaufen dieser Meßstation in einer ähnlichen Vorrichtung 60 wieder aufgestapelt.

An der Vorrichtung sind nicht dargestellte Fühler, beispielsweise Lichtschranken, zur Steuerung des Bewegungs und Meßablaufs vorgesehen. Die Meßergebnisse werden an einen Microprozessor oder an einen Personalcomputer zur Auswertung übertragen. Diese können auch den Anfangsbereich der Kraftdehnungskurve linearisieren, was für die Berechnung der Dehnung und daraus abgeleiteter Größen bei vorgespannten Prüfstreifen unerläßlich ist.

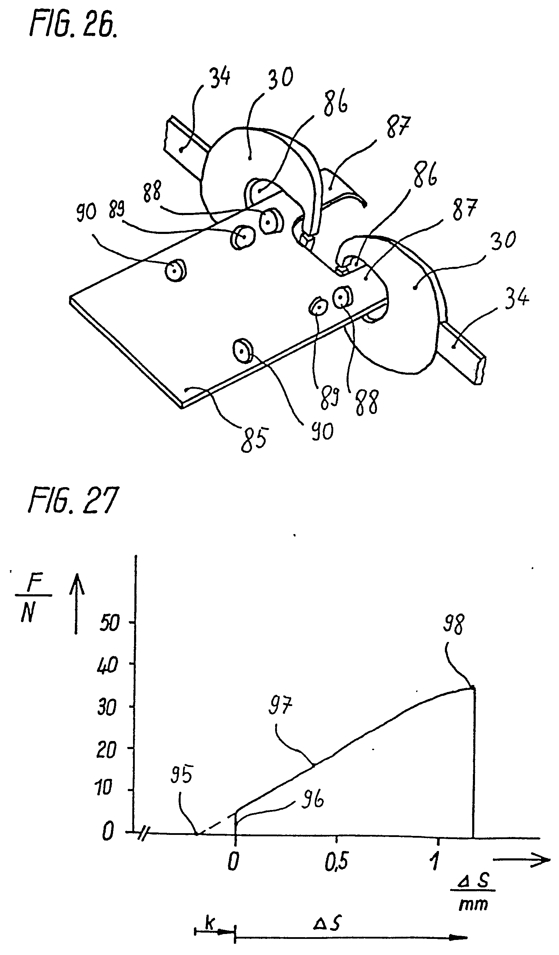

Diese Linearisierungsberechnung ist in Fig. 27 anhand einer Kraft- Dehnungskurve für vorgespannte Prüfstreifen dargestellt. Das steile Kurvenstück 96 entsteht, wenn die Meßklammern die Vorspannkraft von dem Klemmrahmenpaar übernehmen. Das geschieht z.B., wenn die Hauptstreben 5 der Klemmrahmen in Fig. 3 von einem Hebel in Pfeilrichtung gedrückt werden. Die ansteigende Linie 97 entsteht bei Belastung des Prüfstreifens durch die Meßklammern, dessen Bruch am Punkt 98 auftritt.

29

$s_0$ ist die freie Einspannlänge des Prüfstreifens zu Beginn der Messung; $\Delta s$ ist der gemessene Dehnungsweg; k ist die durch lineare Extrapolation 95 gewonnene Korrekturstrecke. Daraus ergibt sich die richtige Berechnung der Bruchdehnung:

$$\varepsilon_0 = \frac{\Delta s + k}{s_0 - k} * 100 \ \%$$

Bei vorgespannten Prüfstreifen ist k positiv, bei zu Beginn der Messung etwas durchhängenden Prüfstreifen ist k negativ.

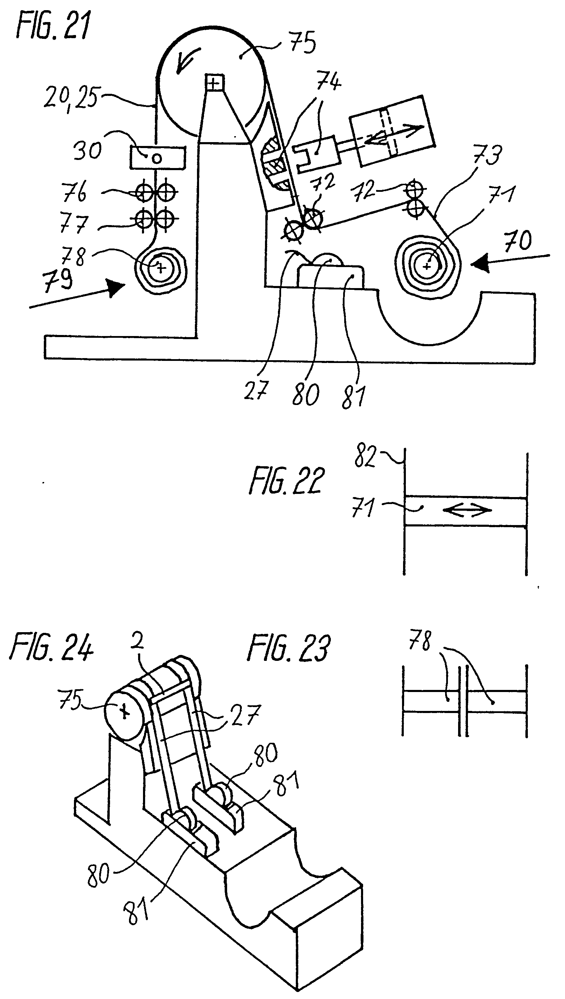

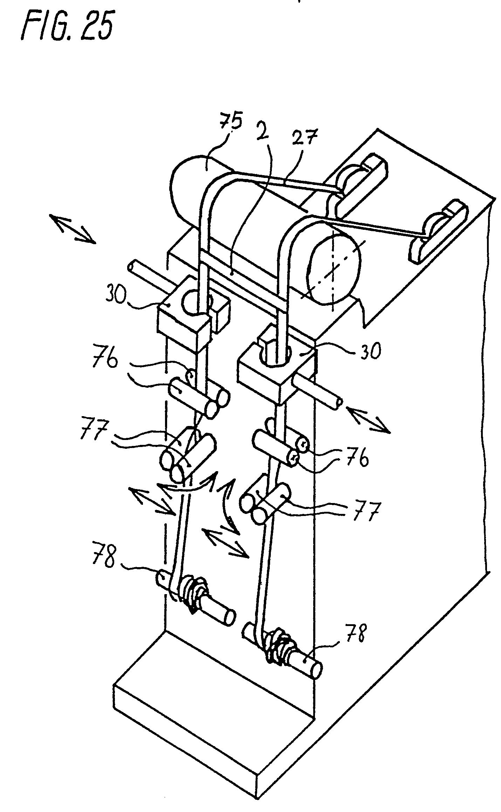

Fig. 21 zeigt ein Prüfgerät, das für lange Probebahnen 73 besonders geeignet ist. Die Probebahn 73 ist auf einer Trommel 71 aufgewickelt. Wie in Fig. 22 dargestellt, ist zur exakten Bahnführung die seitlich verschiebbare Trommel 71 mit Seitenwänden 82 ausgestattet. Die Probebahn 73 wird über Führungsrollen 72 geführt. Das Ausstanzen der Probebahn 73 entsprechend dem Muster nach Fig. 10 erfolgt durch eine im Prüfgerät integrierte Stanze 74. Von einer Trommel 75 wird das Prüfstreifengitter 20, 26 den Meßklammern 30 zugeführt. Unter den Meßklammern 30 sind Zugrollenpaare 76 und 77 und zwei Aufwickelrollen 78 (sh. Fig. 23) angeordnet. Die Zugrollenpaare 76 und 77 und eventuell die Aufwickelrollen 78 ziehen die zerrissenen Teile nach. Die Zugrollenpaare 77 sind um 90° drehbar und senkrecht zu ihren Antriebsachsen verschiebbar, wie dies aus Fig. 25 ersichtlich ist. Dadurch können die Haltestreifen 21 bzw. 27 zentrisch durch die Meßklammern geführt werden. Wenn ein Seitenstreifen 22 bzw. 27 durchläuft, müssen die Zugrollen 77 in ihre Ausgangslage zurückgeschwenkt werden. Werden die Aufwickelrollen 78 nicht gebraucht, z.B. wenn keine Klebstreifen verwendet werden, können die Reste direkt in einen Papierkorb gefüllt werden. An dem Meßgerät befinden sich zwei Halterungen 81 für Klebebandrollen 80, die fluchtend zu den Bahnen der Haltestreifen angeordnet sind. Dadurch ist die Messung von einzelnen Prüfstreifen mit dieser Vorrichtung auch möglich. Die Klebebänder 27 werden, wie in Fig. 24 gezeigt, über die Trommel 75 und durch die Meßklammern 30 zu den Aufwickelrollen 78 geführt. Die Aufwickelrollen spannen die Klebebänder mit möglichst konstanten Kräften, die in den Lagern der Trommel 75 gemessen werden können. Für die Klebebandrollen können Bremsen oder dgl. notwendig sein, die in den Halterungen 81 eingebaut werden können. Die Prüfstreifen 2 werden einfach auf die Klebebänder aufgetragen. Die Regelung der Bahn der Klebebänder 27 bzw. der Haltestreifen 21 und die Steuerung der fluchtenden, rechtwinkligen Ausrichtung der Prüfstreifen 2 in den Meßklammern 30 kann von geeigneten Fühlern, beispielsweise von Lichtschranken, vorgenommen werden.

Fig. 26 zeigt die wichtigsten Teile einer Prüfvorrichtung, die besonders für Handhabungseinheiten nach Fig. 11 geeignet ist.

Dieses Meßgerät hat eine glatte Tischplatte 85, auf die eine vorzugsweise Fig. 11 entsprechende Handhabungseinheit gelegt werden kann. Die Tischplatte 85 hat Fortsetzungsbleche 87, die durch die Meßklammern hindurchragen. Die Meßklammern 30 haben eine für diese Ausführung geeignete, besonders lange lichte Weite 86. Die Bewegungen der Handhabungseinheit werden von gummierten Rollen 88, 89, 90 bewerkstelligt, die auf die entsprechend Fig. 11 besonders breiten Haltestreifen 25 der Handhabungseinheiten einwirken. Die während der Bewegung der Handhabungseinheit in eine Richtung störenden Rollen können während der Bewegung abgehoben werden. Der Vorschub der Handhabungseinheit wird von den Zugrollen 89 bewirkt. Hinter den Meßklammern 30, auf den Fortsetzungsblechen 87 können noch zwei zusätzliche, hier nicht abgebildete Zugrollen angebracht werden. Die Ausrichtung der Handhabungseinheit quer zur Vorschubrichtung und zentrisch zu den inneren lichten Weiten 86 erfolgt mit den angetriebenen Gummirollen 88 und 90. Die Steuerung der Antriebs- und Auflagekräfte der Rollen muß so erfolgen, daß die Handhabungseinheit nicht knickt oder sich aufbiegt und daß sie immer plan auf der Tischplatte aufliegt.

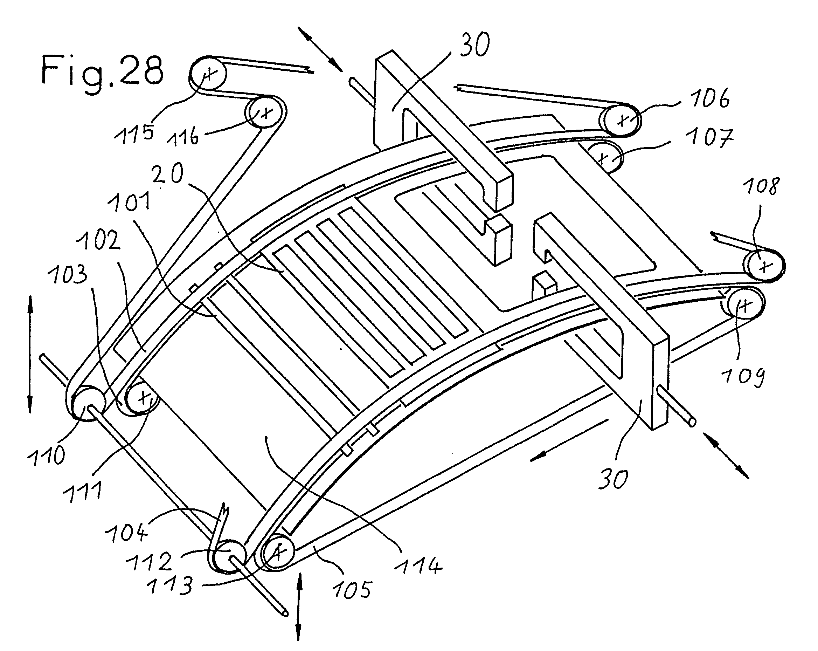

Fig. 28 zeigt ein einfaches Prüfgerät. Es können sowohl einzelne Prüfstreifen 101, als auch eine zusammenhängende Mehrstreifeneinheit 20 verarbeitet werden. Die Prüfstreifen werden von den Bändern 102, 103, 104, 105 gehalten und schrittweise zu den

32

Meßklammern 30 transportiert. Die Bänder werden in Antriebsrollen 106, 107, 108, 109, Umlenkrollen 110, 111, 112, 113 und Spannrollen geführt. Zur Erzeugung einer Haltekraft für Prüfstreifen laufen die Bänder auf einer gekrümmten Abstützplatte 114.

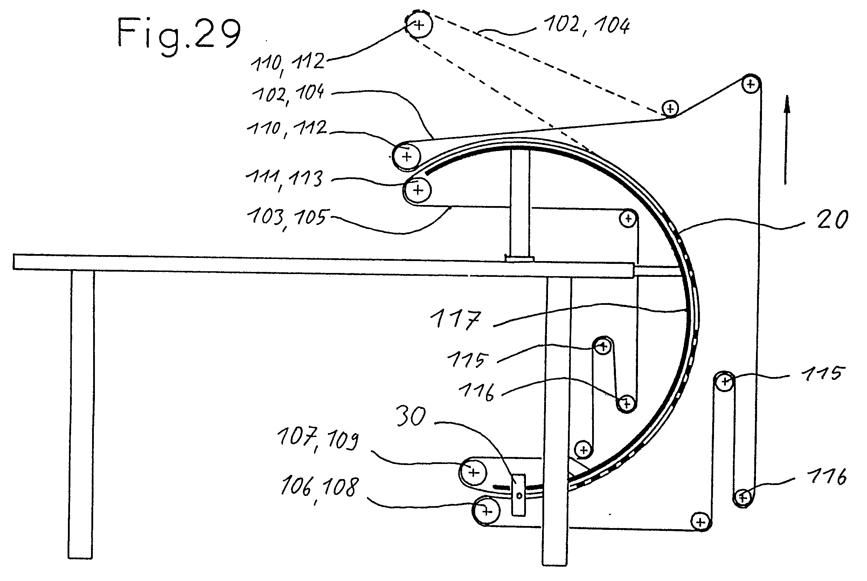

Vor dem Einlegen der Prüfstreifen werden die Bänder 102, 104 mit den Rollen 110, 112 nach oben aufgeklappt. In Fig. 29 ist die aufgeklappte Stellung der Rollen 110, 112 gestrichelt dargestellt.
Die Spannrollen 115, 116 dienen zum gleichmäßigen Spannen der Transportbänder.

Während ein Prüfgerät nach Fig. 28 vorwiegend auf einem Labortisch aufgestellt wird, ist in Fig. 29 ein Prüfgerät darstellt, das seitlich an einem Tisch befestigt ist und dadurch eine längere Ausführung der Abstützplatte 117 und der Transportbänder ermöglicht. Diese Ausführung kann eine größere Anzahl von Prüfstreifen gleichzeitig aufnehmen.

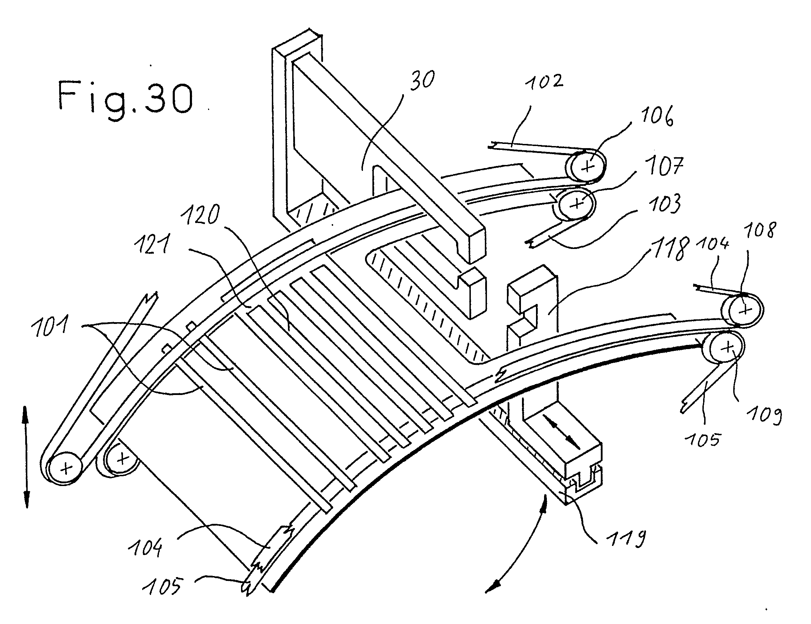

Fig. 30 zeigt eine Ausführungsform für Materialien, die einen langen Dehnungsweg erfordern. Dazu wird eine Meßklammer 30 mit innerer lichter Weite und eine Meßklammer 118 mit Umgehungsteil verwendet. Beide Meßklammern sind auf einem Meßgruppenträger 119 befestigt, wobei nur eine beweglich gelagert werden muß. Wenn die Kraftmeßdose an der Meßklammer 30 angebracht ist, spielt es keine Rolle wie groß

33

die Reibungskräfte für die bewegliche Meßklammer 118 sind. Der Meßgruppenträger 119 ist schwenkbar, wobei der Drehpunkt in der Nähe der Einspannstelle für Prüfstreifen von Meßklammer 30 sein sollte. Zur Messung wird der Meßgruppenträger 119 nach unten ausgeschwenkt, wobei ein Prüfstreifenende aus den Transportbändern 104, 105 herausgezogen wird.

Für diesen Prüfgerätetyp eignen sich einzelne Prüfstreifen 101 oder vorgefertigte Mehrstreifeneinheiten 120, die mit nur einem, einseitigen Haltestreifen 121 ausgeführt sind.

Marek, Konrad, 8938 Buchloe

<u>Verfahren, Prüfstreifen und Vorrichtung zur Prüfung</u>
<u>der Zugfestigkeit von Papier, Karton, Pappe u.ä.</u>

Patentansprüche:

1.   Verfahren zum Prüfen der Zugfestigkeit von Papier, Pappe, Kunststoffolie o.ä., bei welchem Prüfstreifen vorgeschriebener Abmessungen zwischen zwei gegenüberstehend angeordneten Meßklammern eingebracht, in diesen festgeklemmt, einer Zugbeanspruchung unterworfen und die zerrissenen Proben wieder ausgebracht werden, dadurch gekennzeichnet, daß eine Mehrzahl von Prüfstreifen (2) zu einer Einheit (1, 20, 26) zusammengefaßt und schrittweise den Meßklammern (30, 45, 118) zugeführt wird.

2.   Prüfstreifen zum Durchführen des Verfahrens nach Anspruch 1, dadurch gekennzeichnet, daß eine Mehrzahl von Prüfstreifen (2) mit ihren Längsmittellinien parallel zueinander angeordnet und zumindest an ihrem einen Kopfende miteinander zu einer Mehrstreifeneinheit (1, 20, 26) verbunden ist.

3. Mehrstreifeneinheit nach Anspruch 2, dadurch gekennzeichnet, daß die Prüfstreifen (2) nur durch Schlitze oder Schnitte voneinander getrennt sind.

4. Mehrstreifeneinheit nach Anspruch 2, dadurch gekennzeichnet, daß die Prüfstreifen (2) im Abstand voneinander angeordnet sind, daß sie parallel zueinander ausgerichtet sind und daß sie nur an ihren beiden Kopfenden (18, 19) miteinander verbunden sind.

5. Mehrstreifeneinheit nach Anspruch 4, dadurch gekennzeichnet, daß die mehreren Prüfstreifen (2) aus einem einzigen Materialbogen derart herausgestanzt ausgebildet sind, daß die Enden über einen Haltestreifen (21, 25) miteinander verbunden sind.

6. Mehrstreifeneinheit nach Anspruch 2, dadurch gekennzeichnet, daß mehrere einzelne Prüfstreifen (101) oder eine Mehrstreifeneinheit nach zumindest einem der Ansprüche 2 bis 5 zumindest an ihrem einen Ende zwischen zwei Bändern (102, 103 bzw. 104, 105) oder dgl. festgeklemmt werden, wobei die Bänder ebenso wie die Enden der Prüfstreifen außerhalb der Zugbelastungszone der Prüfstreifen liegen.

7. Mehrstreifeneinheit nach Anspruch 4, dadurch gekennzeichnet, daß die Mehrstreifeneinheit (26) aus mit Klebebändern an den Enden (18, 19) zusammengefaßten einzelnen Prüfstreifen (2) gebildet ist.

8. Mehrstreifeneinheit nach Anspruch 4, dadurch gekennzeichnet, daß die Mehrstreifeneinheit (1) aus einer zwischen zwei übereinander angeordneten und mit einer Klammereinrichtung (12) verbindbaren Klemmrahmen (3, 4) an den Enden festgehaltenen Mehrzahl von Prüfstreifen (2) zusammengesetzt ist.

9. Klemmrahmen nach Anspruch 8, dadurch gekennzeichnet, daß eine die Streifenenden fassende Klemmeinrichtung (14) zum vorgespannten Festhalten der mehreren Streifen ausgelegt ist.

10. Klemmrahmen nach Anspruch 8, dadurch gekennzeichnet, daß mindestens der zu messende Prüfstreifen von einem vorgespannten in einen entspannten Zustand überführt werden kann.

11. Meßklammern zum Durchführen eines Zugfestigkeitsprüfverfahrens bei dem eine Mehrzahl von Prüfstreifen in irgend einer Weise zu einer Einheit zusammengefaßt und schrittweise den Meßklammern zugeführt wird, dadurch gekennzeichnet, daß die Meßklammern (30) eine innere lichte Weite (31) aufweisen und daß die Verbindungs- bzw. Haltestreifen (21, 25, 27) der Mehrstreifeneinheiten (20, 26), die Hauptstreben (5) der Klemmrahmenpaare (3, 4), die Transportbänder (102, 103, 104, 105) und/oder die Fortsetzungsteile (87) der Abstützplatte bzw. die Abstützschienen, oder auch nur die Enden der Prüfstreifen ohne anzustoßen durch diese innere lichte Weite hindurchführbar sind.

12. Meßklammern zum Durchführen eines Zugfestigkeitsprüfverfahrens bei dem eine Mehrzahl von Prüfstreifen in irgend einer Weise zu einer Einheit zusammengefaßt und schrittweise den Meßklammern zugeführt wird, dadurch gekennzeichnet, daß die Zugstrebe (44) der Meßklammer (45, 118) gekrümmt ausgeführt ist bzw mit einem Umgehungsteil ausgestattet ist, das die Verbindungs- bzw. Haltestreifen (21, 25, 27)der Mehrstreifeneinheiten (20, 26), die Hauptstreben (5) der Klemmrahmenpaare (3, 4), die Transportbänder (102, 103, 104, 105) und/oder die Fortsetzungsteile (87) der Abstützplatte bzw. die Abstützschienen, oder auch nur die Enden der Prüfstreifen überspannt, ohne anzustoßen.

13. Vorrichtung zum Durchführen des Verfahrens nach Anspruch 1, unter Verwendung von Prüfstreifen nach mindestens einem der Ansprüche 2 bis 10 sowie von Meßklammern nach Anspruch 11, dadurch gekennzeichnet, daß eine Transporteinrichtung vorgesehen ist zum schrittweisen Transport der Mehrstreifeneinheiten (1,20, 26) in einer Richtung im wesentlichen senkrecht zur Verbindungslinie der Meßklammern(30).

14. Vorrichtungen zum Durchführen des Verfahrens nach Anspruch 1 unter Verwendung von Prüfstreifen nach mindestens einem der Ansprüche 2 bis 10, sowie von Meßklammern nach Anspruch 12, dadurch gekennzeichnet, daß eine Transporteinrichtung vorgesehen ist zum schrittweisen Transport der Mehrstreifeneinheiten (1, 20, 26) in der Folge, daß in einem (ersten) Vorschubschritt einer der Prüfstreifen (2) in die Meßklammern (45) bis zu einem Anschlag (46) eingeführt wird, daß in einem (zweiten) Rückwärtsschritt der gemessene Prüfstreifen (2) wieder herausgeführt wird, und daß in einem (dritten) Schritt eine Bewegung im wesentlichen senkrecht zu den vorherigen und zur Hauptebene der Mehrstreifeneinheit durchgeführt wird, um die Mehrstreifeneinheit von der Meßstation (44, 45, 33) freizubekommen.

15. Vorrichtung nach einem der vorhergehenden Ansprüche 13 ff., dadurch gekennzeichnet, daß die Transporteinrichtung zumindest ein Magazin (50, 58, 70)für die zu Handhabungseinheiten zusammengefaßten Prüfstreifen (2), eine Vereinzelungseinrichtung (60), eine Zuführeinrichtung (59) und ein Magazin (79, 50, 58) zur Aufnahme der geprüften Streifen umfaßt.

16. Vorrichtung zum Durchführen des Verfahrens nach Anspruch 1 unter Verwendung von Prüfstreifen nach mindestens einem der Ansprüche 2 bis 7, dadurch gekennzeichnet, daß eine Abstützplatte (85) für die Mehrstreifeneinheiten vorgesehen ist und daß die Abstützplatte Fortsetzungsbleche (87) hat, die durch die innere lichte Weite (86) der Meßklammern (30) hindurchragen bzw. den Umgehungsteil der Meßklammern (45, 118) kreuzen, ohne diese zu berühren.

17. Vorrichtung zum Durchführen des Verfahrens nach Anspruch 16, unter Verwendung von Meßklammern nach Anspruch 11 und 12 und von Bändern nach Anspruch 6, dadurch gekennzeichnet, daß die Bänder oder dgl. auf der Abstützplatte (85, 114, 117) oder auf Abstützschienen laufen und daß diese Abstützelemente die Bänder (102, 103, 104, 105) auf einer gekrümmten Bahn führen.

18. Vorrichtung zum Durchführen des Verfahrens nach Anspruch 1, mit mindestens einer mit Umgehungsteil ausgestatteten Meßklammer (118) nach Anspruch 12, dadurch gekennzeichnet, daß die Meßklammern einen Prüfstreifen zuerst festklemmen, dann durch eine Dreh- oder Hubbewegung aus der Ebene der Handhabungs- bzw.der Mehrstreifeneinheit (120) herausbringen und messen, wobei den Meßklammern nun ein unbegrenzter Dehnungsweg zur Verfügung steht.

19. Vorrichtung nach Anspruch 15, dadurch gekennzeichnet, daß die Magazine (50) zur nebeneinander und aufrechten Aufnahme von Handhabungseinheiten (1) ausgebildet sind.

20. Vorrichtung nach Anspruch 15, dadurch gekennzeichnet, daß die Magazine (58) zur übereinandergestapelten Aufnahme horizontal liegender Handhabungseinheiten (1) ausgebildet sind.

21. Vorrichtung nach Anspruch 20, dadurch gekennzeichnet, daß die Vereinzelungseinrichtung (60) als jeweils die untere Handhabungseinheit (1) freigebende Greifeinrichtung (61, 62, 63, 64) ausgebildet ist.

22. Vorrichtung nach einem der vorhergehenden Ansprüche 13 ff., dadurch gekennzeichnet, daß die Transporteinrichtung unter anderem eine Magazintrommel (71), Führungsrollen (72), eine Zuführtrommel (75), Zugrollen (76, 77) und eventuell Aufwickelrollen (78) umfaßt.

23. Vorrichtung nach einem der vorhergehenden Ansprüche 13 ff., dadurch gekennzeichnet, daß vor den Meßklammern eine Stanzeinrichtung (74) zum Einbringen der zwischen den Prüfstreifen (2) befindlichen Ausnehmungen vorgesehen ist.

FIG.1

1/15

0160292

# FIG. 2

# FIG. 3

FIG. 4

FIG. 5

FIG. 6.

FIG. 7

FIG. 8

FIG. 9

0160292

FIG. 10

FIG. 11

FIG. 12

FIG. 13

20

34

31

30

34

FIG. 14

6/15

0160292

FIG. 15

30

41

32

30

33

40

FIG. 16.

44

45

46

40

33

FIG. 17

45

2

32

33

5

44

43

42

35

FIG. 18

50

5  54  55  57

51

53

52  3  4  56

FIG. 19.

58

3,4  64  5  67  68

63

62

65

69  61  59  66

8/15

0160292

FIG. 20

**FIG. 21**

75
20,25
30
76
77
78
79
74
72
72
73
71
70
27 80 81

**FIG. 22**

82
71

**FIG. 24**

2
75
27
80
81
80
81

**FIG. 23**

78

FIG. 25

0160292

FIG. 26.

FIG. 27

Fig.28

30
106
107
108
109
115 116
20
101
102
103
110
111
104
112
113
105
114
30

0160292

Fig.29

110, 112
102, 104
102, 104
110, 112
111, 113
103, 105
117
115
116
30
107, 109
106, 108
20
115
116

Fig.30